(19) Europäisches Patentamt
European Patent Office
Office européen des brevets

(11) Numéro de publication: **0 078 756**
**B1**

# (12) FASCICULE DE BREVET EUROPEEN

(45) Date de publication du fascicule du brevet: **12.03.86**

(51) Int. Cl.[4]: **C 07 D 295/12**, C 07 D 317/66, A 61 K 31/495

(21) Numéro de dépôt: **82450014.4**

(22) Date de dépôt: **27.10.82**

(54) **Nouvelles (phényl-4 pipérazinyléthyl)-2 anilines, leur méthode de préparation, ainsi que leur emploi en thérapeutique.**

(30) Priorité: **03.11.81 FR 8120564**

(43) Date de publication de la demande: **11.05.83 Bulletin 83/19**

(45) Mention de la délivrance du brevet: **12.03.86 Bulletin 86/11**

(84) Etats contractants désignés: **BE CH DE GB IT LI LU NL**

(56) Documents cités: **BE - A - 620 235** **CH - A - 398 614**

(73) Titulaire: **SOCIETE CORTIAL S.A., 7 rue de l'Armorique, F-75015 Paris (FR)**

(72) Inventeur: **Pontagnier, Henri, 21 rue Edouard Vaillant, F-33600 Pessac (FR)**
Inventeur: **Creuzet, Marie-Hélène, Résidence Jardin de Gambetta T3, F-33000 Bordeaux (FR)**
Inventeur: **Feniou, Claude, 5 rue du Général Guillomat, F-33600 Pessac (FR)**
Inventeur: **Guichard, Françoise, Les cèdres Parc des Tourelles rue St. Elisabeth, F-33200 Bordeaux (FR)**
Inventeur: **Prat, Gisèle, Hameau de noailles Villa 18, F-33400 Talence (FR)**

(74) Mandataire: **Tajan, Marie-Thérèse, LABORATOIRES SARGET Avenue du Président JF Kennedy, F-33701 Mérignac (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 078 756 B1

**Description**

La présente invention concerne de nouvelles (phényl-4-pipérazinyléthyl)-2-anilines, leur méthode de préparation, ainsi que leur emploi en thérapeutique.

Ces produits ont pour formule générale

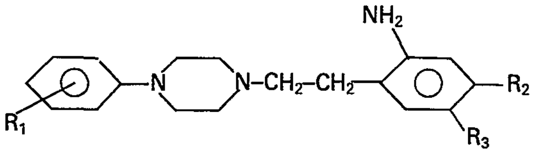

(I)

avec $R_1$ = H ou un ou plusieurs substituants situés en ortho, méta ou para choisis dans le groupe $CH_3$, $CF_3$, F, Cl, $OCH_3$;
$R_2$, $R_3$ identiques ou différents = H, alcoxy inférieur de C1 à C4, $R_2$ et $R_3$ pouvant constituer ensemble une chaine $-O-(CH_2)_n-O-$ avec n = 1 ou 2 ou une chaine $-O-CH_2-O-CH_2-$, sous réserve que $R_1$ soit différent de $CF_3$ si $R_2 = R_3 = H$.

Ces produits peuvent être employés sous forme de base libre ou sous forme de leurs sels pharmacologiquement compatibles tels que les chlorhydrates, citrates, benzilates.

On connaissait jusqu'à présent des dérivés de la (phényl-4-pipérazinyléthyl)-3 ou -4-aniline tels que ceux décrits dans les brevets spéciaux de médicaments n° 191M ou 208M. On connaissait également des dérivés de la (pipérazinyléthyl)-2-aniline substitués sur l'azote 4 du cycle pipérazine par un groupement méthyle (brevet français n° 2476644 ainsi que la ((métatrifluorométhylphényl)-4-pipérazinyléthyl)-2-aniline décrite dans le brevet BE-A-620235. Dans ces deux derniers documents, brevet français n° 2476644 et brevet BE-A-620235, les produits protégés présentent des activités psychotropes du type anxiolytique ou antidépresseur pour le brevet français et non précisées pour le brevet belge. Or nous venons de découvrir que les dérivés de formule (I) présentent de façon tout à fait inattendue des activités antihistaminique et antiallergique permettant leur emploi en thérapeutique antihistaminique et antiallergique.

Les antihistaminiques de synthèse appartiennent principalement aux familles chimiques suivantes: phénothiazine (prométhazine), cycloheptane (cyproheptadine), éthylènediamine (antazoline), aminoéthanol (doxylamine), propylamine (tripolidine), diphénylméthylpipérazine (cinnarizine). Les produits de la présente invention n'appartiennent donc à aucune des familles d'antihistaminiques employés en thérapeutique.

Les produits selon la formule I sont préparés de façon générale par une réaction mettant en jeu un (halogéno-2-éthyl)-2-nitro-1-benzène et une N-phénylpipérazine suivie d'une réduction du dérivé nitré ainsi obtenu.

La présente invention va être décrite de façon plus précise dans les exemples suivants.

Exemple 1

Préparation de la (phényl-4-pipérazinyléthyl)-2-aniline

On chauffe sous agitation au reflux de l'éthanol pendant 15 h le mélange constitué de 0,1 mole de (bromo-2-éthyl)-2-nitro-1-benzène, 0,23 mole de N-phénylpipérazine et 300 $cm^3$ d'éthanol absolu. L'alcool est éliminé par évaporation. Le résidu d'évaporation est repris par de l'eau. On procède à une extraction par l'éther. La phase éthérée est lavée à l'eau, séchée sur $Na_2SO$; l'éther est éliminé par évaporation. L'excès de phénylpipérazine est éliminé par destillation. Le résidu de distillation est recristallisé dans le méthanol. On obtient ainsi le nitro-1-(phényl-4-pipérazinyléthyl)-2-benzène de point de fusion 76°C.

Ce dérivé nitré est dissout dans du méthanol. 7–8 g de Nickel de Raney sont ajoutés à la solution. Le mélange est maintenu sous agitation sous atmosphère d'hydrogène jusqu'à ce que la réaction soit totale. Le Nickel de Raney est éliminé par filtration. Le solvant est éliminé par évaporation.

La (phényl-4-pipérazinyléthyl)-2-aniline est obtenue par cristallisation du résidu d'évaporation dans un mélange d'éther éthylique et d'éther de pétrole. PF 103°C.

Exemple 2

Sont préparés selon la méthode décrite dans l'exemple 1, les dérivés de formule

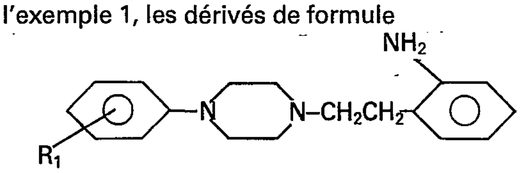

avec

| | |
|---|---|
| $R_1$ = F-4 | PF 113°C |
| $R_1$ = $-OCH_3$-2 | PF 103°C |
| $R_1$ = Cl-4 | PF 126°C |
| $R_1$ = $CH_3$-2 | PF 97°C |

Exemple 3

Préparation de la méthylènedioxy-4,5-((trifluorométhyl-3-phényl)-4-pipérazinyléthyl)-2-aniline

Le mélange constitué de 22,9 g de (chloro-2-éthyl)-6-nitro-5-benzodioxole-1,3, 69 g de N-(trifluorométhyl-3-phényl)-pipérazine et 150 $cm^3$ d'éthanol est chauffé à 120°C sous agitation pendant 18 h. L'éthanol est éliminé par évaporation. Le résidu est repris par de l'éther éthylique et de l'eau. La phase éthérée est séparée par décantation puis extraite par une solution aqueuse d'acide chlorhydrique 2N. Le chlorhydrate du nitro-5-((trifluorométhyl-3-phényl)-4-pipérazinyl-éthyl)-6-benzodioxole-1,3 précipite partiellement. Le précipité filtré et la phase aqueuse acide sont réunis et alcalinisés. Après alcalinisation on procède à une extraction à l'éther éthylique suivie d'un séchage sur $Na_2SO_4$. L'éther est éliminé par évaporation et le résidu est recristallisé dans l'éther éthylique. PF 91°C.

27 g du dérivé nitré ainsi préparé sont mis en suspension dans 1,5 l de méthanol. 7–8 g de Nickel de Raney sont ajoutés. Le mélange est maintenu sous agitation sous atmosphère d'hydrogène jusqu'à ce que la réaction soit totale. Le Nickel de Raney est éliminé par filtration. Le filtrat est évaporé. Le résidu d'évaporation est recristallisé dans un mélange d'éther éthylique et d'éther

de pétrole. On obtient ainsi la méthylènedioxy-4,5-((trifluorométhyl-3-phényl)-4-pipérazinyléthyl)-2-aniline que l'on purifie par recristallisation dans le méthanol. PF 88°C.

On donne dans le tableau ci après les principales caractéristiques des spectres de RMN des produits préparés selon les exemples 1,2 et 3 mis en solution dans $CDCl_3$. Sont indiqués les déplacements chimiques par rapport au TMS pris comme étalon interne, le nombre de protons et la multiplicité des pics avec mc = massif complexe, s = singulet d = dôme.

Les produits faisant l'objet de la présente invention ont subi divers tests pharmacologiques dont nous donnons certains résultats ci-après.

La mortalité induite par les produits de la présente invention a été déterminée chez la souris Swiss exempte d'organismes pathogènes spécifiques. Par voie orale après un taux d'observation de 14 jours, la DL50 du produit de l'exemple 1 administré en solution dans du tween à 20% est de 1175 (946–1459) mg/kg.

L'activité antihistaminique a été déterminée in vitro par la mesure de la concentration entrainant 50% d'inhibition des contractions induites par l'histamine ($1.10^{-8}$ g/l) sur iléon isolé de cobaye (CI50). Cette CI50 est après rinçage de 6,3 (4,14–9,58) $10^{-6}$ g/ml pour le produit de l'exemple 1.

L'activité antiallergique a été déterminé dans le test de l'anaphyllaxie passive cutanée de la façon suivante. On administre à des rats mâles Sprague-Dawley par voie intra-dermique 0,1 ml de sérum riche en IgE. Ce sérum a été fabriqué en sensibilisant des rats Sprague-Dawley par une injection ip d'ovalbumine et de suspension de Bordetella pertussis suivie 20 jours après par une nouvelle injection d'ovalbumine. 24 heures après l'administration du sérum on administre par voie iv 0,5 ml d'une solution contenant 8,25 mg/kg d'ovalbumine et 26,4 mg/kg de bleu d'Evans dans un tampon pH 7,05. Au bout de 30 mn les animaux sont sacrifiés; chaque papule est découpée, pesée et mise à incuber à 37°C pendant 4 j dans 15 g de formamide. La quantité de bleu d'Evans contenue dans chaque papule est déterminée par la mesure de la densité optique du mélange. Le produit à tester est administré par voie orale en suspension homogène en présence de tween 80, 10 mn avant l'injection déclenchante. Son activité est appréciée par le pourcentage de diminution de la quantité de bleu d'Evans ayant diffusé dans la papule. Pour le produit de l'exemple 1 la DE50 est de 5,11 (3,39–7,76) mg/kg.

Compte-tenu de leurs activités antihistaminique et antianaphylactique jointes à une faible toxicité, les produits de la présente invention sont utiles en thérapeutique employés seuls ou en association dans le traitement des états allergiques et anaphylactiques tels que l'urticaire, les prurits, les dermatoses, les eczémas, le rhume des foins, l'oedème de Quincke, la maladie sérique, l'asthme, le choc anaphylactique. Ils pourront être employés également à titre préventif ou curatif dans le traitement du mal des transports.

Ils pourront être administrés par voie orale sous forme par exemple de dragées, comprimés, sirop, ampoules, par voie rectale sous forme de suppositoire, par voie intramusculaire ou intraveineuse ou par voie topique sous forme de pommade ou de gel. Les doses administrées varieront selon l'indication et le sujet de 2 à 200 mg/j en 2 à 6 prises pour la voie orale, de 2 à 200 mg/j en 1 ou 2 prises pour la voie rectale, ou de 0,5 à 50 mg par injection pour les voies parentérales.

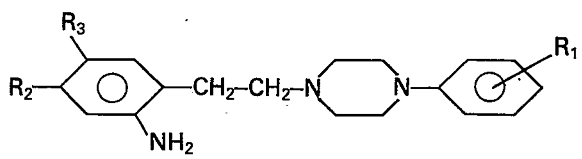

| $R_1$ | $R_2$, $R_3$ | $-CH_2-$ | $NH_2$ | protons aromatiques | divers |
|---|---|---|---|---|---|
| H | H, H | 2,5–3,3ppm;12H;mc | 3,9ppm;2H;d | 6,4–7,4ppm;9H;mc | |
| $CH_3$-2 | H, H | 2,4–3,2ppm;12H;mc | 3,9ppm;2H;d | 6,4–7,3ppm;8H;mc | 2,2ppm;3H;s $CH_3$-C |
| *Cl-4 | H, H | 2,5–3,4ppm;12H;mc | 4,2ppm;2H;d | 6,5–7,4ppm;8H;mc | |
| $OCH_3$-2 | H, H | 2,5–3,3ppm;12H;mc | 4,0ppm;2H;d | 6,5–7,2ppm;8H;mc | 3,8ppm;3H;s $CH_3$-O |
| F-4 | H, H | 2,5–3,3ppm;12H;mc | 3,9ppm;2H;d | 6,5–7,2ppm;8H;mc | |
| $CF_3$-3 | $-O-CH_2-O-$ | 2,4–3,4ppm;12H;mc ($CH_2CH_2$–N) 5,8ppm;2H;s ($-O-CH_2-O-$) | 3,7ppm;2H;d | 6,2 et 6,5ppm;2H;s (H du cycle aniline) 6,9–7,5ppm;4H;mc (H du cycle phénylpipérazine) | |

*Solvant $CDCl_3$ + 3 gouttes de DMSO D6

**Revendications**

1. (Phényl-4-pipérazinyléthyl)-2-anilines de formule générale

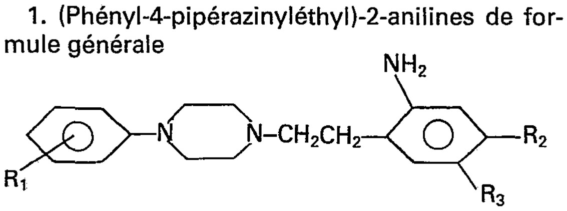

ainsi que leurs sels d'addition non toxiques avec $R_1$ = H ou un ou plusieurs substituants situés en ortho, méta ou para choisi dans le groupe $CH_3$, $CF_3$, F, Cl, $OCH_3$; $R_2$, $R_3$ identiques ou différents = H, alcoxy inférieur de C1 à C4, $R_2$ et $R_3$ pouvant constituer ensemble une chaine $-O-(CH_2)_n-O-$ avec n = 1 ou 2 ou $-OCH_2OCH_2-$ sous réserve que $R_1$ soit différent de $CF_3$ en méta dans le cas où $R_2 = R_3$ = H.

2. Produits selon la revendication 1, caractérisés en ce que $R_2 = R_3$ = H.

3. Produits selon la revendication 1, caractérisés en ce que $R_2$ et $R_3$ forment ensemble une chaine $-O-CH_2-O-$.

4. Procédé de préparation des produits selon les revendications 1 et 3 caractérisé en ce que l'on fait réagir un (halogéno-2-éthyl)-2-nitro-1-benzène et une N-phénylpipérazine et en ce que le dérivé nitré ainsi obtenu est réduit.

5. Médicament utile dans le traitement des états allergiques, caractérisé en ce que le principe actif est constitué par un produit selon l'une des revendications 1 à 3.

6. Composition pharmaceutique ou vétérinaire, caractérisée en ce qu'elle contient à titre de principe actif au moins un des produits selon les revendications 1 à 3 associé avec un véhicule pharmaceutique ou un excipient approprié.

**Patentansprüche**

1- (Phenyl-4-piperazinylethyl)-2-aniline der allgemeinen Formel

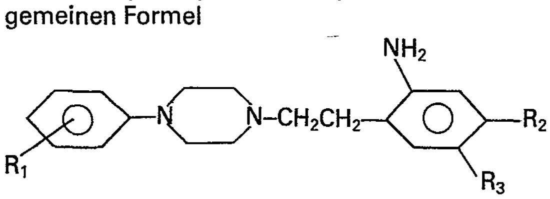

in der

$R_1$ = H oder einen in o-, m- oder p-Stellung befindlichen Substituenten, ausgewählt aus der von $CH_3$, $CF_3$, F, Cl und $OCH_3$ gebildeten Gruppe oder mehrere dieser Substituenten, und

$R_2$ und $R_3$, die gleich oder verschieden sein können, = H oder niederes Alkoxy mit 1–4 Kohlenstoffatomen, oder $R_2$ und $R_3$ zusammen eine Kette $-O-(CH_2)_n-O-$ mit n = 1 oder 2 oder $-OCH_2OCH_2-$, mit der Massgabe, dass $R_1$ unterschiedlich von $CF_3$ in m-Stellung ist, wenn $R_2 = R_3$ = H ist, bedeuten sowie ihre nicht-toxischen Additionssalze.

2. Verbindungen nach Anspruch 1, dadurch gekennzeichnet, dass $R_2 = R_3$ = H ist.

3. Verbindungen nach Anspruch 1, dadurch gekennzeichnet, dass $R_2$ und $R_3$ zusammen eine Kette $-O-CH_2-O-$ bilden.

4. Verfahren zur Herstellung der Verbindungen nach einem der Ansprüche 1 bis 3, dadurch gekennzeichnet, dass man ein (Halogen-2-ethyl)-2-nitro-1-benzol und N-Phenylpiperazin umsetzt und das so erhaltene nitrierte Derivat reduziert.

5. Medikament für die Behandlung von allergischen Zuständen, dadurch gekennzeichnet, dass es als Wirkstoff eine Verbindung nach einem der Ansprüche 1 bis 3 enthält.

6. Pharmazeutische oder veterinärmedizinische Zusammensetzung, dadurch gekennzeichnet, dass sie als Wirkstoff mindestens eines der Produkte nach einem der Ansprüche 1 bis 3 zusammen mit einem pharmazeutischen Träger und einem geeigneten Exzipienten enthält.

**Claims**

1. (Phenyl-4-piperazinethyl)-2-anilines with the general formula

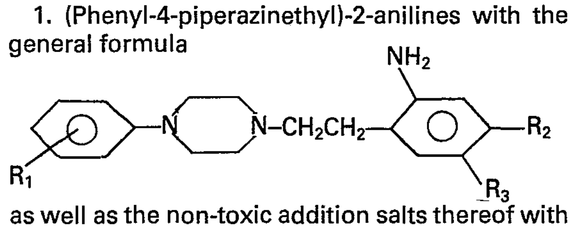

as well as the non-toxic addition salts thereof with $R_1$ = H or one or a plurality of substituents located in ortho, meta, or para selected from the group $CH_3$, $CF_3$, F, Cl, $OCH_3$; $R_2$, $R_3$ being identical or different = H, lower alcoxy from C1 to C4, $R_2$ and $R_3$ may comprise together a chain $-O-(CH_2)_n$ with n = 1 or 2 or $-OCH_2OCH_2-$ under the condition that $R_1$ be different from $CF_3$ in meta in the case where $R_2 = R_3$ = H.

2. Products as defined in claim 1, in which $R_2 = R_3$ = H.

3. Products as defined in claim 1, in which $R_2$ and $R_3$ form together a chain $-O-CH_2-O-$.

4. Method for preparing products as defined in claims 1 and 3, which comprises reacting a (halogeno-2-ethyl)-2-nitro-1 benzene and a N-phenylpiperazine, and reducing the resulting nitrated derivate.

5. Medicament useful for treating allergic conditions, in which the active constituent is comprised of a product as defined in any one of claims 1 to 3.

6. Pharmaceutic or veterinary compound, which comprises as active constituent, at least one of the products defined in claims 1 to 3 in association with a suitable pharmaceutic vehicle or excipient.